(19) Europäisches Patentamt
European Patent Office
Office européen des brevets

(11) Veröffentlichungsnummer : **0 123 838**
**B1**

(12) # EUROPÄISCHE PATENTSCHRIFT

(45) Veröffentlichungstag der Patentschrift : **22.01.86**

(51) Int. Cl.$^4$ : **C 07 C 93/06**, C 07 D295/08, C 07 D211/44, A 61 K 31/13

(21) Anmeldenummer : **84102634.7**

(22) Anmeldetag : **10.03.84**

(54) **Neue Aminopropanolderivate von 1-(2-Hydroxyphenyl)-3-phenylpropanolen, Verfahren zu ihrer Herstellung und diese enthaltende therapeutische Mittel.**

(30) Priorität : **17.03.83 DE 3309595**

(43) Veröffentlichungstag der Anmeldung : **07.11.84 Patentblatt 84/45**

(45) Bekanntmachung des Hinweises auf die Patenterteilung : **22.01.86 Patentblatt 86/04**

(84) Benannte Vertragsstaaten : **AT BE CH DE FR GB IT LI NL SE**

(56) Entgegenhaltungen : **DE-A- 2 001 431**

(73) Patentinhaber : **BASF Aktiengesellschaft**
**Carl-Bosch-Strasse 38**
**D-6700 Ludwigshafen (DE)**

(72) Erfinder : **Franke, Albrecht, Dr.**
**Mandelring 11**
**D-6706 Wachenheim (DE)**
Erfinder : **Gries, Josef, Dr.**
**Roemerweg 43**
**D-6706 Wachenheim (DE)**
Erfinder : **Mueller, Claus D., Dr.**
**Odenwaldring 84**
**D-6806 Viernheim (DE)**
Erfinder : **Lenke, Dieter, Prof. Dr.**
**Kekuleplatz 1**
**D-6700 Ludwigshafen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 123 838 B1

Jouve, 18, rue St-Denis, 75001 Paris, France

**Beschreibung**

Gegenstand der vorliegenden Erfindung sind neue Aminopropanolderivate von 1-(2-Hydroxyphenyl)-3-phenylpropanolen und ihre physiologisch verträglichen Säureadditionsverbindungen, ihr Herstellungsverfahren sowie diese enthaltende therapeutische Mittel, die als Antiarrhythmika verwendet werden können.

Aus der DE-OS 20 01 431 ist bekannt, daß die n-Propylamino-, n-Butyl-amino-, sec.-Butylamino- und tert.-Butylamino-propanolderivate des 2-Hydroxy-β-phenyl-propiophenons antiarrhythmisch wirksam sind. Das gilt besonders für das 2-(2'-Hydroxy-3'-n-propylamino-propoxy)-β-phenyl-propiophenon-hydrochlorid, das unter der Bezeichnung Propafenon als Antiarrhythmikum bekannt ist. Weitere, dem Propafenon strukturell ähnliche Verbindungen sind aus der DE-OS 32 26 863 und der DE-OS 31 33 814 bekannt.

Aufgabe der Erfindung ist es, demgegenüber verbesserte Antiarrhythmika zur Verfügung zu stellen.

Es wurde nun gefunden, daß Aminopropanolderivate von 1-(2-Hydroxyphenyl)-3-phenylpropanolen der Formel I

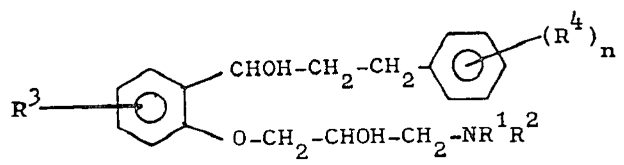

(I)

in der

$R^1$ und $R^2$ gleich oder verschieden sind und Wasserstoffatome, Alkyl-, Cycloalkyl-, Alkenyl-, Alkinyl- oder Hydroxyalkylmethylreste mit jeweils bis zu 6 C-Atomen, Alkoxyalkylmethyl-, Alkylthioalkylmethyl- oder Dialkylaminoalkylmethylreste mit jeweils bis zu 9 C-Atomen oder Phenylalkyl- oder Phenoxyalkylreste mit bis zu 6 C-Atomen im Alkyl und gegebenenfalls im Phenylrest durch Alkyl oder Alkoxy mit jeweils bis zu 3 C-Atomen substituiert bedeuten oder

$R^1$ und $R^2$ zusammen mit dem sie verbindenden Stickstoffatom einen 5- bis 7-gliedrigen gesättigten heterocyclischen Ring bilden, der gegebenenfalls durch einen oder zwei Phenyl- und/oder Hydroxyreste substituiert sein und ein Sauerstoff- oder Stickstoffatom als weiteres Heteroatom im Ring enthalten kann, wobei ein zusätzliches Stickstoffatom durch einen Alkylrest mit 1 bis 3 C-Atomen oder einen Phenylrest substituiert sein kann,

$R^3$ ein Wasserstoffatom, einen Alkylrest mit bis zu 3 C-Atomen, ein Fluor-, Chlor- oder Bromatom, eine Hydroxygruppe oder eine Alkoxygruppe mit bis zu 6 C-Atomen ist,

$R^4$ ein Wasserstoffatom, einen Alkylrest mit bis zu 3 C-Atomen, ein Fluor-, Chlor- oder Bromatom, eine Hydroxygruppe, einen Alkoxyrest mit bis zu 3 C-Atomen oder den Rest $NR^5R^6$ bedeutet, worin $R^5$ und $R^6$ gleich oder verschieden sind und Alkylreste mit bis zu 6 C-Atomen oder zusammen mit dem sie verbindenden Stickstoffatom einen heterocyclischen Ring darstellen, und

n die Zahl 1 oder 2 ist,

und ihre physiologisch verträglichen Säureadditionssalze wertvolle pharmakologische Eigenschaften aufweisen.

Von den Verbindungen der Formel I sind diejenigen besonders hervorzuheben, in denen $R^3$ Wasserstoff bedeutet. Die Gruppe $NR^1R^2$ ist vorzugsweise ein Piperidin-, Piperazin-, N-Methylpiperazin-, Morpholin- oder Diisopropylaminorest. Weiter sind für $R^1$ und $R^2$ insbesondere Wasserstoff, Propyl-, Butyl-, β-Alkoxyalkyl und β-Hydroxyalkylreste zu nennen, wie n-Propyl, Isopropyl, n-Butyl, Isobutyl, tert.-Butyl, n-Pentyl, sec.-Pentyl, Isopentyl, Neopentyl, β-Methoxyethyl und Hydroxyethyl. $R^4$ ist vorzugsweise ein Wasserstoffatom, eine Hydroxy-, $C_1$- bis $C_4$-Alkoxy-, eine Dimethylamino- oder Diethylaminogruppe.

Außer den in den Beispielen angegebenen Verbindungen seien beispielhaft genannt :

1-[2-(2'-Hydroxy-3'-isopropylamino-propoxy)-phenyl]-3-(4'-dimethylaminophenyl)-propanol,
1-[2-(2'-Hydroxy-3'-n-butylamino-propoxy)-phenyl]-3-(4'-dimethylaminophenyl)-propanol,
1-[2-(2'-Hydroxy-3'-tert.-butylamino-propoxy)-phenyl]-3-(4'-dimethylaminophenyl)-propanol,
1-[2-(2'-Hydroxy-3'-(1,1-dimethylpropylamino)-propoxy)-phenyl]-3-(4'-dimethylaminophenyl)-propanol,
1-[2-(2'-Hydroxy-3'-n-propylamino-propoxy)-phenyl]-3-(3'-dimethylaminophenyl)-propanol,
1-[2-(2'-Hydroxy-3'-(1,1-dimethylpropylamino)-propoxy)-phenyl]-3-(3'-dimethylaminophenyl)-propanol,
1-[2-(2'-Hydroxy-3'-n-propylamino-propoxy)-phenyl]-3-(4'-diethylaminophenyl)-propanol,
1-[2-(2'-Hydroxy-3'-piperidino-propoxy)-phenyl]-3-(4'-dimethylaminophenyl)-propanol,
1-[2-(2'-Hydroxy-3'-diisopropylamino-propoxy)-phenyl]-3-(4'-dimethylaminophenyl)-propanol,
1-[2-(2'-Hydroxy-3'-n-propylamino-propoxy)-5-hydroxy-phenyl]-3-phenylpropanol,
1-[2-(2'-Hydroxy-3'-n-propylamino-propoxy)-4-hydroxy-phenyl]-3-phenylpropanol,
1-[2-(2'-Hydroxy-3'-n-propylamino-propoxy)-5-methoxy-phenyl)-3-phenylpropanol,
1-[2-(2-Hydroxy-3'-n-propylamino-propoxy)-4-methoxy-phenyl)-3-phenylpropanol.

Die erfindungsgemäßen Verbindungen können hergestellt werden, indem man die entsprechenden Aminopropanolderivate von 2-Hydroxy-β-phenyl-propiophenonen der Formel II

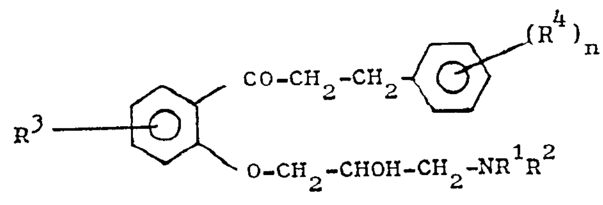

(II)

reduziert.

Die Reduktion kann durch katalytische Reduktion oder durch Umsetzung mit komplexen Metallhydriden durchgeführt werden.

Für die katalytische Reduktion mit Wasserstoff kommen Katalysatoren wie $PtO_2$, Raney-Nickel, Pd-C oder Pd-$BaSO_4$ in Betracht. Bevorzugt werden Raney-Nickel und $PtO_2$ eingesetzt, wobei vorzugsweise unter neutralen oder basischen Bedingungen gearbeitet wird und die Reduktion nach Aufnahme eines Äquivalentes Wasserstoff abgebrochen wird, um eine Weiterreduktion zu vermeiden. Die Hydrierung kann unter Normaldruck oder unter erhöhtem Druck in einem geschlossenen Druckgefäß durchgeführt werden, bevorzugt ist die drucklose Hydrierung. Die Reduktion kann in einem Temperaturbereich zwischen Raumtemperatur und 100 °C durchgeführt werden, bevorzugt wird bei 20 bis 30 °C gearbeitet. Als Lösungsmittel für die Reduktion mit Wasserstoff kommen in erster Linie Alkohole wie Methanol, Ethanol oder Isopropanol in Betracht. Für die Reduktion mit komplexen Metallhydriden eignen sich beispielsweise $NaBH_4$, $LiAlH_4$, Diboran, Diboran/$BF_3$, Lithiumboranat, $Na[BH_3CN]$, $KBH_4$ oder Natrium-bis-(2-methoxy-ethoxy)-dihydridoaluminat in einem protischen oder aprotischen Lösungsmittel. Bevorzugte Ausführungsformen sind die Reduktion mit $NaBH_4$ in Methanol/$H_2O$ oder Ethanol/$H_2O$ bei Temperaturen zwischen 20 und 50 °C oder mit $LiAlH_4$ in absolutem Ether, Dioxan oder Tetrahydrofuran bei Temperaturen zwischen 20 °C und der Rückflußtemperatur des Lösungsmittels.

Die benötigten Ausgangsverbindungen der Formel II sind bekannt (vgl. DE-OS 20 01 431, 31 33 814 und 32 26 863). Sie lassen sich herstellen aus den entsprechenden 2-Hydroxy-β-phenyl-propiophenonen durch Umsetzung mit Epichlor- oder Epibromhydrin zum Glycidether und anschließende nucleophile Ringöffnung mit den entsprechenden Aminen.

Die erfindungsgemäßen Verbindungen der Formel I besitzen am Kohlenstoffatom 2 der aliphatischen Seitenketten und am Kohlenstoffatom 1 des 1,3-Diphenylpropanols je ein Chiralitätszentrum und werden in der Regel als Diastereomerengemisch erhalten. Durch fraktionierte Kristallisation lassen sich die Diastereomerenpaare voneinander trennen, die Racemate können nach bekannten Methoden, beispielsweise durch Bildung diastereomerer Salze mit optisch aktiven Hilfssäuren, wie Dibenzoylweinsäure, Campher-10-sulfonsäure, Ditoluylweinsäure oder 3-Bromcampher-8-sulfonsäure, in die optisch aktiven Antipoden getrennt werden.

Gegebenenfalls werden die erhaltenen erfindungsgemäßen Verbindungen in das Säureadditionssalz einer physiologisch verträglichen Säure überführt. Als übliche physiologisch verträgliche anorganische Säuren kommen beispielsweise in Betracht: Salzsäure, Bromwasserstoffsäure, Phosphorsäure oder Schwefelsäure, und als organische Säuren beispielsweise Oxalsäure, Maleinsäure, Fumarsäure, Milchsäure, Weinsäure, Äpfelsäure, Zitronensäure, Salicylsäure, Adipinsäure oder Benzoesäure. Weitere Säuren können aus Fortschritte der Arzneimittelforschung Band 10, Seiten 224 bis 225, Birkhäuser Verlag, Basel und Stuttgart, 1966, entnommen werden.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol wie Methanol, Ethanol oder Propanol, oder einem niederen Keton, wie Aceton, Methylethylketon oder Methylisobutylketon, oder einem Ether, wie Diethylether, Tetrahydrofuran oder Dioxan, erhalten. Zur besseren Kristallabscheidung können auch Mischungen der genannten Lösungsmittel verwendet werden. Darüber hinaus können pharmazeutisch vertretbare wäßrige Lösungen von Säure-Additionsverbindungen der Aminopropanolderivate der Formel I durch Auflösen der freien Base in einer wäßrigen Säurelösung hergestellt werden.

Die erfindungsgemäßen Verbindungen und ihre physiologisch verträglichen Säureadditionssalze sind wegen ihrer antiarrhythmischen, β-sympatholytischen und Ca-antagonistischen Eigenschaften insbesondere zur Pharmakotherapie von Herzrhythmusstörungen und zur Prophylaxe des plötzlichen Herztodes sowie zur Behandlung der coronaren Herzkrankheit geeignet.

Zur Untersuchung der pharmakodynamischen Eigenschaften der erfindungsgemäßen Produkte wurde folgende Methode verwendet:

Die Substanzen wurden Ratten (Stamm: Sprague-Dawley, Gewicht 200 bis 250 g) oral appliziert. 45 min später wurden die Tiere mit Thiobutabarbital-Natrium (100 mg/kg i.p.) narkotisiert. Als arrhythmogene Substanz wurde Aconitin 60 min nach Substanzapplikation i.v. infundiert (Dosierungsgeschwindigkeit : 0,005 mg/kg pro min). Bei unbehandelten Tieren (N = 52) traten nach 2,74 ± 0,07 min im EKG Arrhythmien auf, deren Eintritt durch Antiarrhythmica dosisabhängig verzögert wurde.

Aus der linearen Beziehung zwischen log Dosis (mg/kg) der Prüfsubstanzen und der relativen Verlängerung der Aconitininfusionsdauer Δ % wurde die Dosis bestimmt, welche die Infusionsdauer um 50 % verlängerte (ED 50 %).

Ferner wurde aus der in den Experimenten verwendeten dezimalgeometrischen Dosenfolge (Faktor $\sqrt[3]{10}$) die Dosis ermittelt, bei welcher toxische Symptome (Veränderungen im Ausgangs-EKG, Cyanose, Krämpfe) auftraten. Als Maß für die therapeutische Breite der neuen Verbindungen wurde der Quotient aus akuter toxischer Dosis und antiarrhythmischer ED 50 % bestimmt.

Die erfindungsgemäßen Verbindungen sind bei oraler Applikation in Dosen unter 46,4 mg/kg antiarrhythmisch, β-sympatholytisch und Ca-antagonistisch wirksam. Sie übertreffen damit die Vergleichssubstanz Propafenon [2-(2-Hydroxy-3-propylamino-propoxy)-3-phenylpropiophenonhydrochlorid] eindeutig.

Die neuen Verbindungen können in üblicher Weise oral oder intravenös verabfolgt werden. Die Dosierung hängt vom Alter, Zustand und Gewicht des Patienten sowie von der Applikationsart ab. In der Regel beträgt die tägliche Wirkstoffdosis zwischen etwa 5 und 75 mg/kg Körpergewicht bei oraler Gabe und zwischen etwa 1 und 10 mg/kg Körpergewicht bei parenteraler Gabe.

Die neuen Verbindungen können in den gebräuchlichen galenischen Applikationsformen fest oder flüssig angewendet werden, z. B. als Tabletten, Filmtabletten, Kapseln, Pulver, Granulate, Dragees, Suppositorien oder Lösungen gen. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln und/oder Antioxidantien verarbeitet werden (vgl. H. Sucker et al : Pharmazeutische Technologie, Thieme-Verlag, Stuttgart, 1978). Die so erhaltenen Applikationsformen enthalten den Wirkstoff normalerweise in einer Menge von 1 bis 99 Gewichtsprozent.

Die folgenden Beispiele sollen die Erfindung näher erläutern :

A. Herstellung der Ausgangsverbindungen

Beispiel I

2-(2'-3'-Epoxypropoxy)-5-chlor-β-phenyl-propiophenon

69 g (0,265 mol) 2-Hydroxy-5-chlor-β-phenyl-propiophenon wurden mit 125,4 g (0,915 mol) Epibromhydrin, 70 ml Dimethylformamid und 48,7 g (0,353 mol) wasserfreiem $K_2CO_3$ 5 h bei 60 °C gut gerührt. Nach dem Abkühlen wurde mit 250 ml $H_2O$ versetzt und die organische Phase durch Destillation unter vermindertem Druck vom überschüssigen Epibromhydrin befreit. Der verbleibende Rückstand wurde aus Cyclohexan/Methyl-tert.-butylether umkristallisiert.

Ausbeute : 67 g (79 %) ; Fp. 46 bis 47 °C.

Beispiel II

2-(2'-Hydroxy-3'-n-propylamino-propoxy)-5-chlor-β-phenyl-propiophenonhydrochlorid

5 g (0,016 Mol) 2-(2',3'-Epoxypropoxy)-5-chlor-β-phenyl-propiophenon und 5 g (0,08 Mol) n-Propylamin wurden in 100 ml Isopropanol gelöst und 8 h auf dem Wasserbad gehalten. Nach dem Erkalten wurden das Lösungsmittel und das überschüssige Amin unter vermindertem Druck abdestilliert ; es verblieben 5,6 g öliger Rückstand, aus dem mit Hilfe von etherischer Salzsäure das Hydrochlorid hergestellt und aus Aceton/Ether umkristallisiert wurde.

Ausbeute : 4,7 g (71 %), Fp : 163 bis 164 °C.

B. Herstellung der erfindungsgemäßen Verbindungen

Beispiel 1

1-[2-(2'-Hydroxy-3'-n-propylamino-propoxy)-5-chlor-phenyl]-3-phenylpropanol-oxalat

6 g 2-(2'-Hydroxy-3'-n-propylamino-propoxy)-5-chlor-β-phenyl-propiophenon (0,014 Mol) wurden in 300 ml Ethanol/$H_2O$ (~ 3 : 1) gelöst und portionsweise mit 800 mg $NaBH_4$ versetzt. Die Lösung wurde 8 h bei Raumtemperatur stehen gelassen. Anschließend wurde mit 500 ml $H_2O$ versetzt und mehrere Male mit Ether extrahiert. Die vereinigten Etherextrakte wurden getrocknet und der Ether unter vermindertem Druck abdestilliert ; es verblieben 5,5 g öliger Rückstand. Der Rückstand wurde in Ethanol aufgenommen, mit der berechneten Menge Oxalsäure versetzt und bis zur Trübung mit Essigester versetzt. Nach mehrtägigem Stehen und Einengen der Lösung wurde das Produkt nach dem Trocknen als amorphes weißes Pulver vom Fp. 55 bis 64 °C isoliert.

Beispiel 2

1-[2-(2'-Hydroxy-3'-n-propylamino-propoxy)-phenyl]-3-phenyl-propanol

10 g (0,03 Mol) 2-(2'-Hydroxy-3'-n-propylamino-propoxy)-β-phenyl-propiophenon wurden in 300 ml Ethanol gelöst und unter gutem Rühren in Gegenwart von 2 g Pd/C bei Normaldruck bei einer Temperatur von 25 bis 40 °C hydriert. Nach 24 h war die Wasserstoffaufnahme beendet. Anschließend wurde vom Katalysator abfiltriert und das Lösungsmittel unter vermindertem Druck abdestilliert. Der verbleibende Rückstand wurde aus Ligroin unter Zusatz von wenig Aceton umkristallisiert. Es wurden so 4,8 g (48 %) 1-[2-(2'-Hydroxy-3-n-propylamino-propoxy)-phenyl]-3-phenyl-propanol isoliert, Fp. 86 bis 87 °C.

Analog Beispiel 1 wurden hergestellt :

3. 1-[2-(2'-Hydroxy-3'-diisopropylamino-propoxy)-phenyl]-3-phenylpropanol-tartrat, Fp : 118 °C
4. 1-[2-(2'-Hydroxy-3'-piperidino-propoxy)-phenyl]-3-phenyl-propanoloxalat, Fp : 153 °C
5. 1-[2-(2'-Hydroxy-3'-(3-methyl-1-butin-3-ylamino)-propoxy)-phenyl]-3-phenyl-propanol-oxalat, Fp : 149 °C
6. 1-[2-(2'-Hydroxy-3'-(1-butin-3-ylamino)-propoxy)-phenyl]-3-phenyl-propanol-oxalat, Fp : 173 °C
7. 1-[2-(2'-Hydroxy-3'-(3-methoxyprop-2-ylamino)-propoxy)-phenyl]-3-phenyl-propanol-oxalat, Fp : 153 °C
8. 1-[2-(2'-Hydroxy-3'-(N-methyl-N-(2-(3,4-dimethoxy-phenyl)-ethylamino))-propoxy)-phenyl]-3-phenyl-propanol-oxalat, Fp : 72 °C
9. 1-[2-(2'-Hydroxy-3'-(4-hydroxy-4-phenyl-piperidino)-propoxy)-phenyl]-3-phenyl-propanol, Fp : 79 °C
10. 1-[2-(2'-Hydroxy-3'-(1,1-dimethyl-propylamino)-propoxy]-phenyl]-3-phenyl-propanol-tartrat, amorph
11. 1-[2-(2'-Hydroxy-3'-isopropylamino-propoxy)-phenyl]-3-phenyl-propanoltartrat, amorph
12. 1-[2-(2'-Hydroxy-3'-tert.-butylamino-propoxy)-phenyl]-3-phenyl-propanol-tartrat, amorph
13. 1-[2-(2'-Hydroxy-3'-sec.-butylamino-propoxy)-phenyl]-3-phenyl-propanol-tartrat, amorph
14. 1-[2-(2'-Hydroxy-3'-n-propylamino-propoxy)-phenyl]-3-(2-methoxy-phenyl)-propanol-oxalat, Fp : 81 bis 95 °C
15. 1-[2-(2'-Hydroxy-3'-n-propylamino-propoxy)-phenyl]-3-(4-methylphenyl)-propanol-oxalat, Fp : 146 °C
16. 1-[2-(2'-Hydroxy-3'-n-propylamino-propoxy)-phenyl]-3-(4-chlorphenyl)-propanol-oxalat, Fp : 151 bis 154 °C
17. 1-[2-(2'-Hydroxy-3'-n-propylamino-propoxy)-phenyl]-3-(4-dimethylaminophenyl)-propanol-bishydrochlorid, Fp : 50 bis 60 °C
18. 1-[2-(2'-Hydroxy-3'-n-propylamino-propoxy)-5-methyl-phenyl]-3-(3,4-dimethoxyphenyl)-propanol-oxalat, Fp : 136 °C
19. 1-[2-(2'-Hydroxy-3'-n-propylamino-propoxy)-phenyl]-3-(3,4-di-methoxyphenyl)-propanol-oxalat, Fp : 139 bis 141 °C
20. 1-[2-(2'-Hydroxy-3'-n-propylamino-propoxy)-5-methylphenyl]-3-(4-chlorphenyl)-propanol-oxalat, Fp : 173 °C

**Patentansprüche** (für die Vertragsstaaten : BE, CH, DE, FR, GB, IT, LI, NL, SE)

1. Aminopropanolderivate von 1-(2-Hydroxyphenyl)-3-phenylpropanolen der Formel I

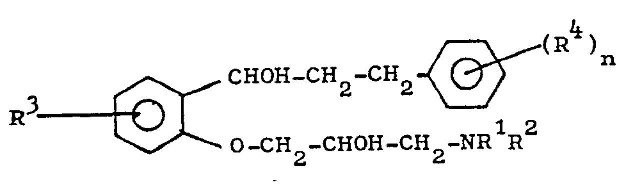

(I)

in der

$R^1$ und $R^2$ gleich oder verschieden sind und Wasserstoffatome, Alkyl-, Cycloalkyl-, Alkenyl-, Alkinyl- oder Hydroxyalkylmethylreste mit jeweils bis zu 6 C-Atomen, Alkoxyalkylmethyl-, Alkylthioalkylmethyl- oder Dialkylaminoalkylmethylreste mit jeweils bis zu 9 C-Atomen oder Phenylalkyl- oder Phenoxyalkylreste mit bis zu 6 C-Atomen im Alkyl und gegebenenfalls im Phenylrest durch Alkyl oder Alkoxy mit jeweils bis zu 3 C-Atomen substituiert bedeuten oder

$R^1$ und $R^2$ zusammen mit dem sie verbindenden Stickstoffatom einen 5- bis 7-gliedrigen gesättigten heterocyclischen Ring bilden, der gegebenenfalls durch einen oder zwei Phenyl- und/oder Hydroxyreste substituiert sein und ein Sauerstoff- oder Stickstoffatom als weiteres Heteroatom im Ring enthalten kann, wobei ein zusätzliches Stickstoffatom durch einen Alkylrest mit 1 bis 3 C-Atomen oder einen Phenylrest substituiert sein kann,

$R^3$ ein Wasserstoffatom, einen Alkylrest mit bis zu 3 C-Atomen, ein Fluor-, Chlor- oder Bromatom, eine Hydroxygruppe oder eine Alkoxygruppe mit bis zu 6 C-Atomen ist,

$R^4$ ein Wasserstoffatom, einen Alkylrest mit bis zu 3 C-Atomen, ein Fluor-, Chlor- oder Bromatom, eine Hydroxygruppe, einen Alkoxyrest mit bis zu 3 C-Atomen oder den Rest $NR^5R^6$ bedeutet, worin $R^5$ und $R^6$ gleich oder verschieden sind und Alkylreste mit bis zu 6 C-Atomen oder zusammen mit dem sie verbindenden Stickstoffatom einen heterocyclischen Ring darstellen, und

n die Zahl 1 oder 2 ist,

und ihre physiologisch verträglichen Säureadditionssalze.

2. Verfahren zur Herstellung der Verbindungen der Formel I gemäß Anspruch 1, dadurch gekennzeichnet, daß man die entsprechenden Aminopropanolderivate von 2-Hydroxy-β-phenyl-propiophenonen der Formel II

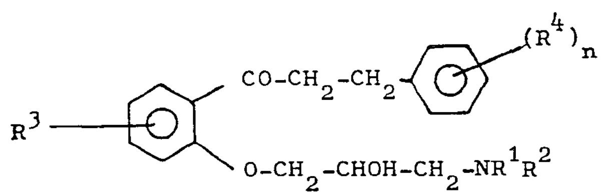

(II)

reduziert.

3. Verbindungen der Formel I gemäß Anspruch 1 zur Verwendung bei der Bekämpfung von Krankheiten.

4. Verwendung der Verbindungen der Formel I gemäß Anspruch 1 zur Herstellung eines Arzneimittels zur Bekämpfung von Herzkrankheiten.

**Patentanspruch** (für den Vertragsstaat AT)

Verfahren zur Herstellung von Aminopropanolderivaten von 1-(2-Hydroxyphenyl)-3-phenylpropanolen der Formel I

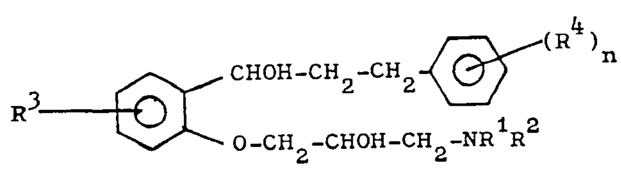

(I)

in der

$R^1$ und $R^2$ gleich oder verschieden sind und Wasserstoffatome, Alkyl-, Cycloalkyl-, Alkenyl-, Alkinyl- oder Hydroxyalkylmethylreste mit jeweils bis zu 6 C-Atomen, Alkoxyalkylmethyl-, Alkylthioalkylmethyl- oder Dialkylaminoalkylmethylreste mit jeweils bis zu 9 C-Atomen oder Phenylalkyl- oder Phenoxyalkylreste mit bis zu 6 C-Atomen im Alkyl und gegebenenfalls im Phenylrest durch Alkyl oder Alkoxy mit jeweils bis zu 3 C-Atomen substituiert bedeuten oder

$R^1$ und $R^2$ zusammen mit dem sie verbindenden Stickstoffatom einen 5- bis 7-gliedrigen gesättigten heterocyclischen Ring bilden, der gegebenenfalls durch einen oder zwei Phenyl- und/oder Hydroxyreste substituiert sein und ein Sauerstoff- oder Stickstoffatom als weiteres Heteroatom im Ring enthalten kann, wobei ein zusätzliches Stickstoffatom durch einen Alkylrest mit 1 bis 3 C-Atomen oder einen Phenylrest substituiert sein kann,

$R^3$ ein Wasserstoffatom, einen Alkylrest mit bis zu 3 C-Atomen, ein Fluor-, Chlor- oder Bromatom, eine Hydroxygruppe oder eine Alkoxygruppe mit bis zu 6-Atomen ist,

$R^4$ ein Wasserstoffatom, einen Alkylrest mit bis zu 3 C-Atomen, ein Fluor-, Chlor- oder Bromatom, eine Hydroxygruppe, einen Alkoxyrest mit bis zu 3 C-Atomen oder den Rest $NR^5R^6$ bedeutet, worin $R^5$ und $R^6$ gleich oder verschieden sind und Alkylreste mit bis zu 6 C-Atomen oder zusammen mit dem sie verbindenden Stickstoffatom einen heterocyclischen Ring darstellen, und

n die Zahl 1 oder 2 ist,

und deren Salze mit physiologisch verträglichen Säure, dadurch gekennzeichnet, daß man die entsprechenden Aminopropanolderivate von 2-Hydroxy-β-phenyl-propiophenonen der Formel II

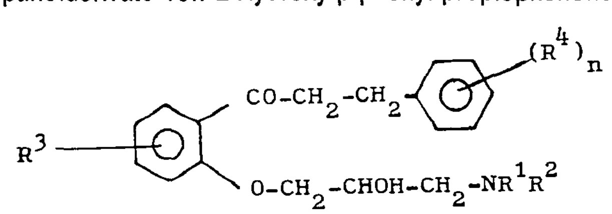

(II)

reduziert.

**Claims** (for the Contracting States : BE, CH, DE, FR, GB, IT, LI, NL, SE)

1. An aminopropanol derivative of a 1-(2-hydroxyphenyl)-3-phenylpropanol of the formula I

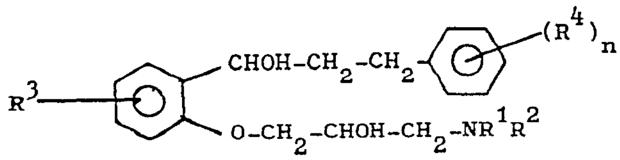

(I)

where

$R^1$ and $R^2$ are identical or different and are each hydrogen, alkyl, cycloalkyl, alkenyl, alkynyl or hydroxyalkylmethyl, each of up to 6 carbon atoms, alkoxyalkylmethyl, alkylthioalkylmethyl or dialkylaminoalkylmethyl, each of up to 9 carbon atoms, or a phenylalkyl or phenoxyalkyl radical where alkyl is of up to 6 carbon atoms and the phenyl radical is unsubstituted or substituted by alkyl or alkoxy, each of up to 3 carbon atoms, or

$R^1$ and $R^2$, together with the nitrogen atom to which they are bonded, form a 5-membered to 7-membered saturated heterocyclic ring which can be unsubstituted or monosubstituted or disubstituted by phenyl and/or hydroxyl and can contain an oxygen or nitrogen atom as a further hetero atom in the ring, and an additional nitrogen atom may be substituted by alkyl of 1 to 3 carbon atoms or by phenyl,

$R^3$ is hydrogen, alkyl of up to 3 carbon atoms, fluorine, chlorine, bromine, hydroxyl or alkoxy of up to 6 carbon atoms,

$R^4$ is hydrogen, alkyl of up to 3 carbon atoms, fluorine, chlorine, bromine, hydroxyl, alkoxy of up to 3 carbon atoms or a radical $NR^5R^6$, where $R^5$ and $R^6$ are identical or different and are each alkyl of up to 6 carbon atoms or, together with the nitrogen atom to which they are bonded, form a heterocyclic ring, and

n is 1 or 2,

and its physiologically tolerated addition salts with acids.

2. A process for the preparation of a compound of the formula I as claimed in claim 1, wherein the corresponding aminopropanol derivative of a 2-hydroxy-β-phenylpropiophenone of the formula II

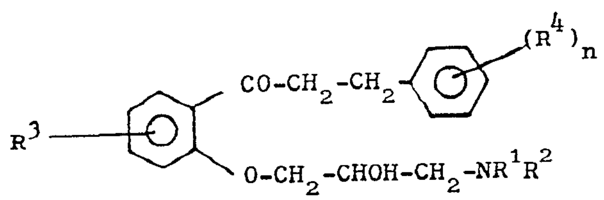

(II)

is reduced.

3. A compound of the formula I as claimed in claim 1 for use in the treatment of disorders.

4. The use of a compound of the formula I as claimed in claim 1 for the preparation of a drug for treating cardiac disorders.

**Claim** (for the Contracting State AT)

A process for the preparation of an aminopropanol derivative of a 1-(2-hydroxyphenyl)-3-phenylpropanol of the formula I

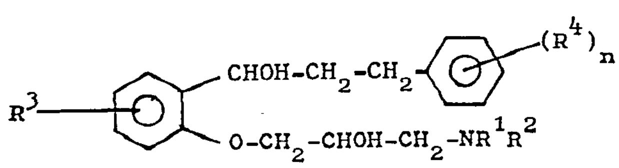

(I)

where

$R^1$ and $R^2$ are identical or different and are each hydrogen, alkyl, cycloalkyl, alkenyl, alkynyl or hydroxyalkylmethyl, each of up to 6 carbon atoms, alkoxyalkylmethyl, alkylthioalkylmethyl or dialkylaminoalkylmethyl, each of up to 9 carbon atoms, or a phenylalkyl or phenoxyalkyl radical where alkyl is of up to 6 carbon atoms and the phenyl radical is unsubstituted or substituted by alkyl or alkoxy, each of up to 3 carbon atoms, or

$R^1$ and $R^2$, together with the nitrogen atom to which they are bonded, form a 5-membered to 7-membered saturated heterocyclic ring which can be unsubstituted or monosubstituted or disubstituted

by phenyl and/or hydroxyl and can obtain an oxygen or nitrogen atom as a further hetero atom in the ring, and an additional nitrogen atom may be substituted by alkyl of 1 to 3 carbon atoms or by phenyl,

$R^3$ is hydrogen, alkyl of up to 3 carbon atoms, fluorine, chlorine, bromine, hydroxyl or alkoxy of up to 6 carbon atoms,

$R^4$ is hydrogen, alkyl of up to 3 carbon atoms, fluorine, chlorine, bromine, hydroxyl, alkoxy of up to 3 carbon atoms or a radical $NR^5R^6$, where $R^5$ and $R^6$ are identical or different and are each alkyl of up to 6 carbon atoms or, together with the nitrogen atom to which they are bonded, form a heterocyclic ring, and

n is 1 or 2,

and its physiologically tolerated addition salts with acids, wherein the corresponding aminopropanol derivative of a 2-hydroxy-β-phenylpropiophenone of the formula II

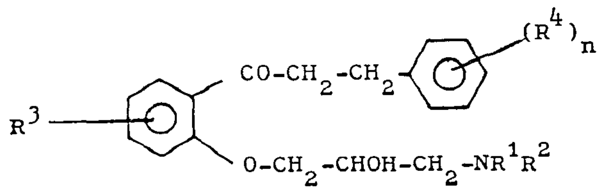

(II)

is reduced.

**Revendications** (pour les Etats contractants : BE, CH, DE, FR, GB, IT, LI, NL, SE)

1. Dérivés aminopropanols de 1-(2-hydroxyphényl)-3-phényl-propanols de formule I

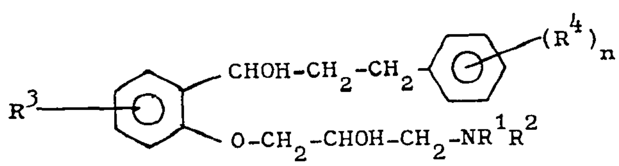

(I)

dans laquelle

$R^1$ et $R^2$ sont identiques ou différents et représentent des atomes d'hydrogène, des restes alkyle, cycloalkyle, alcényle, alcinyle ou hydroxyalkylméthyle ayant respectivement jusqu'à 6 atomes C, des restes alcoxyalkylméthyle, alkylthioalkylméthyle ou dialkylaminoalkylméthyle ayant respectivement jusqu'à 9 atomes C ou des restes phénylalkyle ou phénoxyalkyle ayant jusqu'à 6 atomes C dans alkyle et éventuellement substitués, dans le reste phényle, par alkyle ou alcoxy ayant respectivement jusqu'à 3 atomes C ou

$R^1$ et $R^2$ forment, avec l'atome d'azote qui les relient, un noyau hétérocyclique saturé à 5 à 7 chaînons, qui est éventuellement substitué par un ou deux reste phényle et/ou hydroxy et qui peut contenir, comme autre hétéroatome dans le noyau, un atome d'oxygène ou d'azote, un atome d'azote additionnel pouvant être substitué par un reste alkyle ayant 1 à 3 atomes C ou par un reste phényle,

$R^3$ est un atome d'hydrogène, un reste alkyle ayant jusqu'à 3 atomes C, un atome de fluor, chlore ou brome, un groupe hydroxy ou un groupe alcoxy ayant jusqu'à 6 atomes C,

$R^4$ représente un atome d'hydrogène, un reste alkyle ayant jusqu'à 3 atomes C, un atome de fluor, chlore ou brome, un groupe hydroxy, un reste alcoxy ayant jusqu'à 3 atomes C ou le reste $NR^5R^6$ où $R^5$ et $R^6$ sont identiques ou différents et représentent des restes alkyle ayant jusqu'à 6 atomes C ou, avec l'atome d'azote qui les relient, un noyau hétérocyclique, et

n est le nombre 1 ou 2

et leurs sels d'addition d'acide compatibles physiologiquement.

2. Procédé de préparation de composés de formule I selon la revendication 1, caractérisé par le fait qu'on réduit les dérivés aminopropanols de 2-hydroxy-β-phényl-propiophénones de formule II

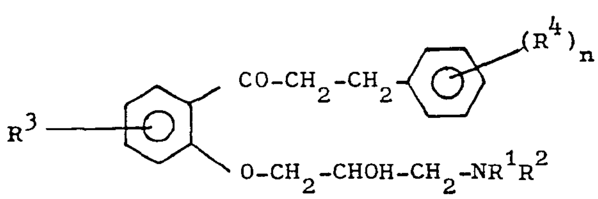

(II)

3. Composés de formule I selon la revendication 1 pour l'utilisation dans la lutte contre des maladies.

4. Utilisation des composés de formule I selon la revendication 1 pour la préparation d'un médicament pour la lutte contre les maladies du cœur.

**Revendication** (pour l'Etat contractant AT)

Procédé de préparation de dérivés d'aminopropanols de 1-(2-hydroxyphényl)-3-phénylpropanols de formule I

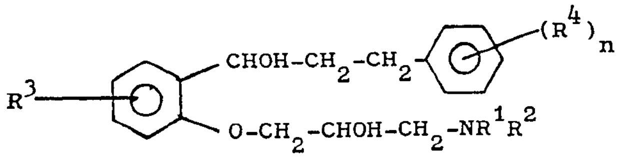

(I)

dans laquelle

$R^1$ et $R^2$ sont identiques ou différents et représentent des atomes d'hydrogène, des restes alkyle, cycloalkyle, alcényle, alcinyle ou hydroxyalkylméthyle ayant respectivement jusqu'à 6 atomes C, des restes alcoxyalkylméthyle, alkylthioalkylméthyle ou dialkylaminoalkylméthyle ayant respectivement jusqu'à 9 atomes C ou des restes phénylalkyle ou phénoxyalkyle ayant jusqu'à 6 atomes C dans alkyle et éventuellement substitués, dans le reste phényle, par alkyle ou alcoxy ayant respectivement jusqu'à 3 atomes C ou

$R^1$ et $R^2$ forment, avec l'atome d'azote qui les relient, un noyau hétérocyclique saturé à 5 à 7 chaînons, qui est éventuellement substitué par un ou deux reste phényle et/ou hydroxy et qui peut contenir, comme autre hétéroatome dans le noyau, un atome d'oxygène ou d'azote, un atome d'azote additionnel pouvant être substitué par un reste alkyle ayant 1 à 3 atomes C ou par un reste phényle,

$R^3$ est un atome d'hydrogène, un reste alkyle ayant jusqu'à 3 atomes C, un atome de fluor, chlore ou brome, un groupe hydroxy ou un groupe alcoxy ayant jusqu'à 6 atomes C,

$R^4$ représente un atome d'hydrogène, un reste alkyle ayant jusqu'à 3 atomes C, un atome de fluor, chlore ou brome, un groupe hydroxy, un reste alcoxy ayant jusqu'à 3 atomes C ou le reste $NR^5R^6$ où $R^5$ et $R^6$ sont identiques ou différents et représentent des restes alkyle ayant jusqu'à 6 atomes C ou, avec l'atome d'azote qui les relient, un noyau hétérocyclique, et

n est le nombre 1 ou 2

et leurs sels d'addition d'acide compatibles physiologiquement, caractérisé par le fait qu'on réduit les dérivés aminopropanols de 2-hydroxy-β-phényl-propiophénones de formule II

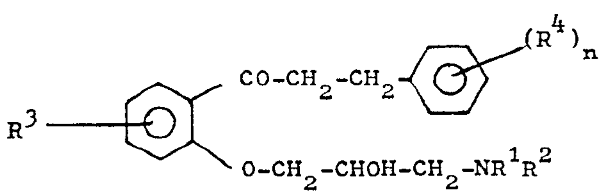

(II)